# EUROPEAN PATENT SPECIFICATION

(11) **EP 0 392 228 B1**
(45) Date of publication and mention of the grant of the patent: **18.11.1993**
(21) Application number: 90105416.3
(22) Date of filing: 22.03.1990
(51) Int. Cl.: A61M 5/50

(54) **Non-reusable syringe**
Einwegspritze
Seringue non réutilisable

(30) Priority: 22.03.1989 AR 313481
(43) Date of publication of application: 17.10.1990
(73) Proprietor: Arcusin, Carlos Eduardo, Buenos Aires (AR)
(72) Inventor: Arcusin, Carlos Eduardo, Buenos Aires (AR)
(74) Representative: Liedl, Gerhard, Dipl.-Phys.

(56) References cited:
- EP-A- 0 300 694
- EP-A- 0 329 358
- WO-A-88/10127
- GB-A- 2 015 883
- US-A- 3 985 122

## Description

The present invention relates to a non-reusable syringe having a tube-shaped body ending in a spout, a movable piston fitting into the cavity of the body, a stem for operating the piston, and means for limiting the run of the piston.

This syringe is intended for use in the field of medicine, amonst others, and can be used only once.

Disposable syringes, intended to be used only once for medical application in order to avoid contagion, are known, but their re-use cannot be prevented, so that a patient cannot be sure that a syringe has not been used previously. There is a need for syringes which can be used only once.

The document GB 2 015 883 A describes a disposable syringe having a cylinder of which one end is formed with a nozzle permitting the fixing of a needle, an intake and delivery piston having a body arranged so as to form a tight movable partition capable of sliding inside the cylinder, a rod fast with the piston body and permitting the piston body to be displaced by a sliding movement in the cylinder, and means preventing the rearward return of at least a part of the piston body, when the body occupies a position close to its position of maximum injection in the cylinder. The means for preventing a rearward return may be formed by a circular groove in which can be engaged a peripheral rim of a resilient member integral with the body of the piston, or by a member such as an O-ring which engages in a groove in the cylinder wall and becomes detached from the rest of the piston if a rearward stroke is attempted. Before the piston body occupies the position of maximum injection in the cylinder, the piston can be made to carry out an indefinite number of reciprocatory movements during the filling of the cylinder before the operation of injection.

The document WO 88/10127 describes a single use syringe with a barrel and a plunger slidably mounted in the barrel with the plunger stopper sealingly engaging with the inner wall of the barrel. Projections extend from the inner wall of the barrel and are located at such a distance from the distal end of the barrel to permit flushing movements of the stopper. A breakable joint joins the stopper and the plunger rod and is broken following an engagement of the stopper with the projections during advance or withdrawal of the plunger, so that the stopper is retained in the end of the barrel and the syringe is rendered unfit for further use.

It is the object of the invention to provide a discardable syringe that can be used only once and does not permit an application of tricks allowing a repeated use.

According to the invention, this object is achieved by the features of patent claim 1.

The syringe of the present invention can be used only once, since just one loading operation with injection-means and only one ejection operation of the injection-means contained therein can be performed by it. The mechanism forming the piston and the stem controlling it, together with the run limiting means that the syringe body has, determine that the piston, after its both ways run inside the syringe body, will stay inoperable, nothwithstanding a displacement of the stem or an intention to bind the stem to the piston by external actions.

In order to remain inoperable, the syringe has its piston formed by two independent and complementary bodies.

One of them is a body that frictions against the internal walls of the syringe along its run, and the other one is an element having a determined geometrical configuration which is connected to the stem and which allows modification of its situation with respect to the the first body when it passes through a hole that the first body has in the middle, and so, the first body may be drawn in one way and pushed only once in the opposite direction. This is possible because one of the two bodies is flexible and the other one is rigid and there exist means to limit the piston's run inside the syringe.

The syringe of this invention is constituted by a tube-shaped body, with a spout in its extremity. A piston fitting perfectly well into the cavity of the body, runs in same. The piston is constituted by two complementary elements. The first element is the one that fits into the cavity of the syringe body, and the second element has a geometrical configuration, selected among truncated conical, cylindrical, semicylindrical, or polygonal affixed to the bar performing the piston's run.

This second element may change its location with respect to the first element when it passes through the hole of said first element having a smaller diameter than that of the second element, since - in a selective way- one of the elements is made of a flexible material and the other of a rigid material, and the syringe body has, in a part of same, limiting means for the run of the piston whose function is to allow the second element - from which the piston is formed - to change its location with respect to the first element.

In order to clarify the advantages so slightly mentioned and to which experts will be able to add many more, and in order to make its comprehension easier with respect to the constructive, constitutive and functional features of this syringe, there is a preferred example described below, as shown in the figures, considering that same has not a limitative character with reference to its scope.

Figure 1 shows a syringe according to the invention.

Figure 2 is a partially cut perspective view.

Figure 3 shows a variant of the piston conformation.

Figure 4 shows another variant of the piston conformation.

Figure 5 shows a cut of the limiting means according to line V-V of figure 1.

Figures 6 and 7 show cuts of variants of the limiting means.

Figures 8, 9, 10, 11 and 12 show the piston positions and its forming elements during the syringe operation.

The syringe shown in the figures is constituted by a body 1 formed by a tube having a spout 2 in its extremity or end. A piston 3 is placed inside of body 1, this being a movable piston.

This piston 3 is formed by a first element 4 having a similar external outline as the internal outline of the tube in body 1 and having in its central part a hole 5 of a diameter slightly bigger than the diameter of a guide-butt 6 provided in the second element 7 which forms piston 3 together with element 4.

The second element 7 has the shape of a truncated cone 8 in one of its realizations, whose smaller base is connected to stem 9 which operates piston 3. The bigger base of the truncated cone 8 is affixed to guide-butt 6. The truncated cone 8 is placed with its smaller conical part introduced in the hole 5 of the element 4 when piston 3 is positioned over the ending of body 1 containing spout 2.

In another realization, as shown in figure 3, the second element has a spherical shape 10 and guide-butt 6 and stem 9 are fixed in diametrically opposite places of said sphere.

Furthermore, as shown in figure 4, the second element 7 is formed by a hemisphere 11 where stem 9 is applied in its spherical part and guide-butt 6 is in the flat part. This second element 7 may also have polygonal geometrical configurations constituting a complementary element to form piston 3.

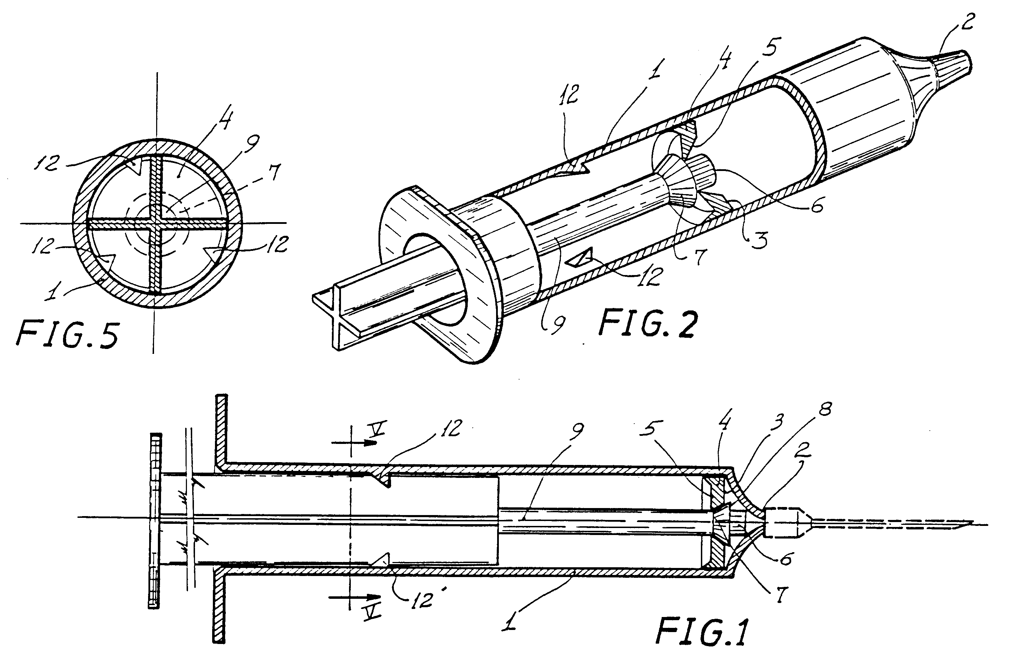

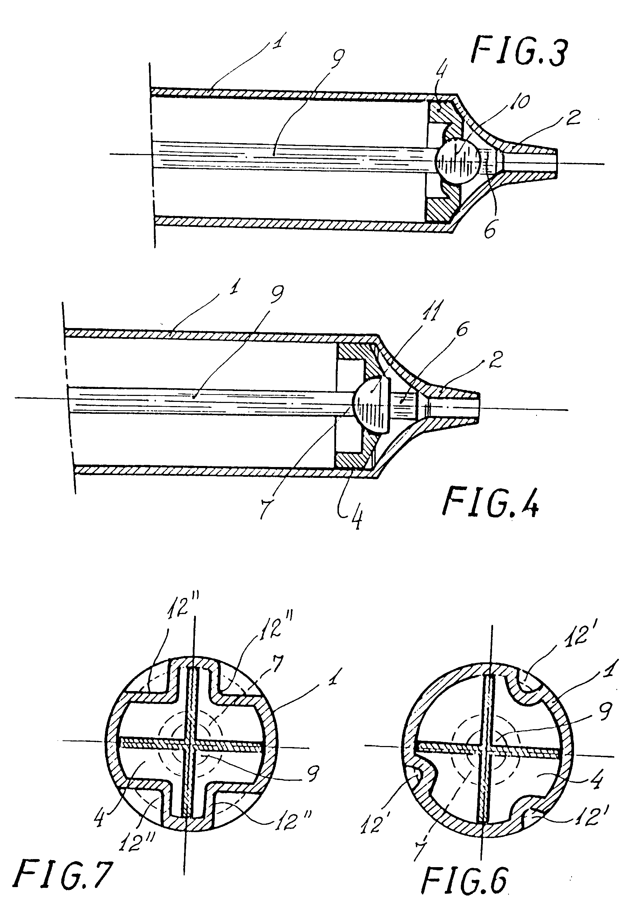

The body 1 of the syringe has limiting means 12 for the run, near its termination. These limiting means may be formed by re-entrants made at 120° among each other, as shown in figure 5, or flattening of the tube of the body 1, as shown in figures 6 and 7.

Stem 9, which operates piston 3, may be cylindrical or have a cross shape with an adequate termination for its use.

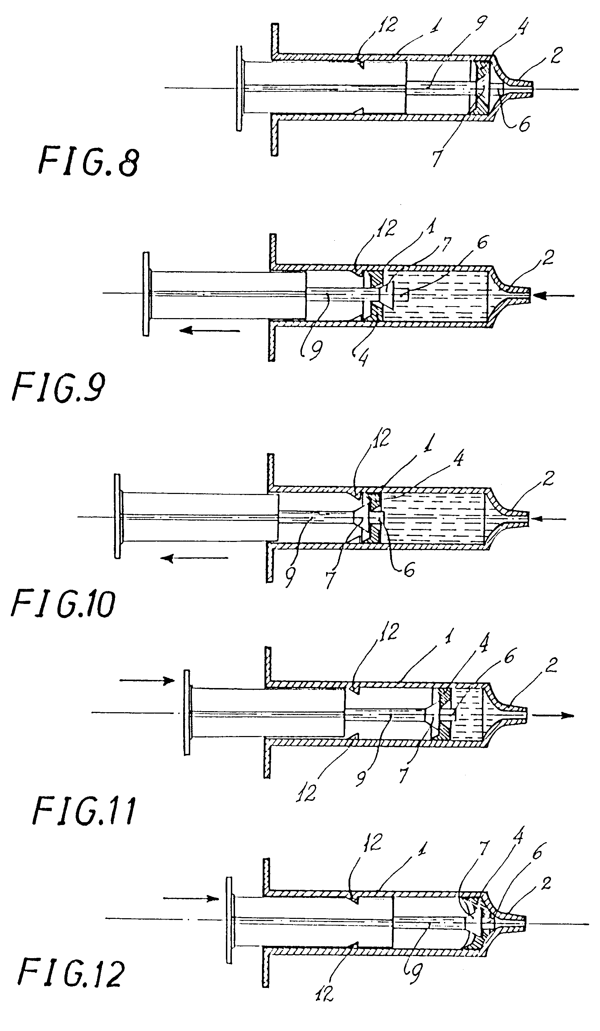

Figures 8 to 12 show the operation of the syringe.

When the piston 3 is placed near spout 2 (fig. 8), which is a necessary condition to fill the syringe, the second element 7 is placed between the termination of body 1 and first element 4. When the piston 3 is retracted by actuating its stem 9 to absorb the injection-means to be introduced into the syringe, element 7 is introduced into hole 5 of element 4 forming a watertight whole, as seen in figure 9.

When piston 3 reaches the run limiting means 12 and if one wishes to eject the means introduced into the syringe, the syringe stem 9 must continue to be operated in the same direction until the second element 7 occupies the position shown in figure 10. This is because one of the elements 4 and 7 is elastic and the other is rigid.

From the position shown in figure 10, it is possible to put pressure on the stem 9 as the group of elements 4 and 7 act like a piston and the means introduced into the syringe can be ejected through the spout 2 (figure 11) up to the end of the run (figure 12). If suction is desired again, the piston 3 does not operate because element 7 will be separated from element 4 because they are not physically joined to each other (fig. 12). Therefore, the syringe cannot be used again.

If the intention is to place element 7 in the position of initial figure 8, this would be impossible because the guide-butt 6 is preventing it, since it would be touching the bottom of the syringe which will not allow element 4 to pass through hole 5.

When the present invention will be taken to practice, no doubt some amendments may be introduced in certain details, without leaving aside the main principles clearly detailed in the claims.

## Claims

1. A non-reusable syringe having
- a tube-shaped body (1) ending in a spout (2),
- a piston (3) fitting into the cavity of the body (1),
- a stem (9) for operating the piston (3), and
- means (12) for limiting the run of the piston (3),
wherein
- the piston (3) is formed by two independent elements (4, 7), these being a first element (4) that fits into the cavity of the syringe body (1) with its external outline against the internal wall of the body (1) and is of an elastic material, and a second element (7) that is connected to the stem (9), that is of a rigid material and that has a geometrical conformation selected among truncated conical, cylindrical, semicylindrical or polygonal,
- the stem (9) and a guide-butt (6) are fixed in opposite positions to the faces of the second element (7), and
- the elastic first element (4) has in its centre a hole (5) therethrough having a smaller diameter than the second element (7), the second element (7) changing its location with respect to the first element (4) when it passes through the hole (5),
the operation of the syringe being such that
- when the piston (3) is near the spout (2), as a condition for filling the syringe, the second element (7) is between the termination of the body (1) and the first element (4),
- when the stem of the piston (3) is actuated to introduce the injection-means into the syringe, the second element (7) is introduced into the hole (5) of the first element (4) to form a watertight whole, and
- when the piston (3) reaches the run limiting means (12) and the stem (9) continues to be operated in the same direction, the second element (7) passes through the hole of the first element (4) so that it occupies a position from which it is possible to put pressure on the stem (9) so that the elements (4 and 7) act like a piston and the injection-means are ejected through the spout (2) up to the end of the run when the guide-butt (6) touches the bottom of the syringe, this not allowing the first element (4) to pass through the hole (5).

2. A syringe according to claim 1, wherein the second element (7) has the shape of a truncated cone (8), the smaller base of which is connected to the stem (9) and the bigger base of which is affixed to the guide-butt (6).

3. A syringe according to claim 1, wherein the second element (7) has a spherical shape (10), and the guide-butt (6) and the stem (9) are fixed in diametrically opposite places of the sphere.

4. A syringe according to claim 1, wherein the second element (7) is formed by a hemisphere (11) where the stem (9) is applied in its spherical part and the guide-butt (6) is in the flat part.

5. A syringe according to claim 1, wherein the second element (7) has polygonal geometric configurations.

6. A syringe according to claim 1, wherein the run limiting means (12) are formed by bosses on the wall of the tube constituting the body (1) of the syringe at 120° between them.

7. A syringe according to claim 6, wherein the run limiting means (12) are formed by flattenings having a curved shape on the walls of the tube.

8. A syringe according to claim 6, wherein the run limiting means (12) are formed by rectangular flattenings on the walls of the tube.

9. A syringe according to claim 6, wherein the run limiting means (12) are formed by a jointly binding bushing to the body (1) of the syringe containing the stem (9).

10. A syringe according to claim 6, wherein the run limiting means (12) are formed by a flattening of the termination of the tube of the syringe.

## Patentansprüche

1. Einwegspritze mit
- einem rohrförmigen Körper (1), der in einer Tülle (2) endet,
- einem Kolben (3), der in den Hohlraum des Körpers (1) eingepaßt ist,
- einem Stiel (9) zum Betätigen des Kolbens (3) und
- Mitteln (12) zum Begrenzen des Hubes des Kolbens (3),
bei der
- der Kolben (3) von zwei unabhängigen Elementen (4, 7) gebildet wird, welche aus einem ersten Element (4), das mit seinem Außenumfang gegen die Innenwand des Körpers (1) in den Hohlraum des Spritzenkörpers (1) eingepaßt und aus einem elastischen Material ist, und einem zweiten Element (7) bestehen, das mit dem Stiel (9) verbunden ist, aus einem starren Material ist und eine geometrische Form aufweist, die ausgewählt ist aus einer stumpfkegeligen, zylindrischen, halbzylindrischen oder polygonalen,
- der Stiel (9) und ein Führungsanschlag (6) an einander gegenüberliegenden Stellen an den Flächen des zweiten Elementes (7) befestigt sind, und
- das elastische erste Element (4) in seiner Mitte eine durchgehende Öffnung (5) aufweist, die einen kleineren Durchmesser als das zweite Element (7) hat, wobei das zweite Element (7) seine Lage bezüglich des ersten Elementes (4) ändert, wenn es sich durch die Öffnung (5) hindurchbewegt,
wobei die Funktionsweise der Spritze derart ist, daß
- wenn, als Bedingung zum Füllen der Spritze, der Kolben (3) sich in der Nähe der Tülle (2) befindet, das zweite Element (7) zwischen dem Ende des Körpers (1) und dem ersten Element (4) befindlich ist,
- wenn der Stiel des Kolbens (3) zum Einführen des Injektionsmittels in die Spritze betätigt wird, das zweite Element (7) zur Bildung einer wasserdichten Einheit in die Öffnung (5) des ersten Elementes (4) eingeführt wird, und
- wenn der Kolben (3) die Hubbegrenzungsmittel (12) erreicht und der Stiel (9) weiterhin in der gleichen Richtung betätigt wird, das zweite Element (7) sich durch die Öffnung des ersten Elements (4) hindurchbewegt, so daß es eine Lage einnimmt, aus der es möglich ist, einen Druck auf den Stiel (9) auszuüben, so daß die Elemente (4 und 7) wie ein Kolben wirken und das Injektionsmittel bis zum Ende des Hubes, an dem der Führungsanschlag (6) den Boden der Spritze berührt, durch die Tülle (2) ausgestoßen wird, wobei hierdurch ein Hindurchbewegen des ersten Elementes (4) durch die Öffnung (5) nicht gestattet wird.

2. Spritze nach Anspruch 1, bei der das zweite Element (7) die Form eines Stumpfkegels (8) aufweist, dessen kleinere Basis mit dem Stiel (9) verbunden und dessen größere Basis an dem Führungsanschlag (6) befestigt ist.

3. Spritze nach Anspruch 1, bei der das zweite Element (7) eine Kugelform (10) aufweist und der Führungsanschlag (6) und der Stiel (7) an diametrisch gegenüberliegenden Stellen der Kugel befestigt sind.

4. Spritze nach Anspruch 1, bei der das zweite Element (7) von einer Halbkugel (11) gebildet wird, wobei der Stiel (9) an seinem kugelförmigen Teil angesetzt ist und der Führungsanschlag (6) sich am flachen Teil befindet.

5. Spritze nach Anspruch 1, bei der das zweite Element (7) polygonale geometrische Formen aufweist.

6. Spritze nach Anspruch 1, bei der die Hubbegrenzungsmittel (12) von Vorsprüngen, mit 120° dazwischenliegend, an der Wand des den Körper (1) darstellenden Rohres gebildet werden.

7. Spritze nach Anspruch 6, bei der die Hubbegrenzungsmittel (12) von Abflachungen mit gekrümmter Form an den Wänden des Rohres gebildet werden.

8. Spritze nach Anspruch 6, bei der die Hubbegrenzungsmittel (12) von rechteckigen Abflachungen an den Wänden des Rohres gebildet werden.

9. Spritze nach Anspruch 6, bei der die Hubbegrenzungsmittel (12) von einer Hülse gebildet werden, die mit dem Körper (1) der den Stiel (9) enthaltenden Spritze verbunden ist.

10. Spritze nach Anspruch 6, bei der die Hubbegrenzungsmittel (12) von einer Abflachung des Endes des Rohres der Spritze gebildet werden.

## Revendications

1. Seringue non réutilisable comportant :
- un corps tubulaire (1) se terminant par un canon (2),
- un piston (3) monté dans la cavité du corps (1),
- une tige (9) pour actionner le piston (3), et
- des moyens (12) pour limiter la course du piston (3), dans laquelle :
- le piston (3) est formé par deux éléments indépendants (4, 7), ces éléments étant un premier élément (4) qui est monté dans la cavité du corps (1) de seringue avec son contour extérieur contre la paroi intérieure du corps (1) et qui est en un matériau élastique, et un second élément (7) qui est fixé à la tige (9), qui est en un matériau rigide et qui a une forme géométrique choisie parmi les formes tronconique, cylindrique. semi-cylindrique ou polygonale,
- la tige (9) et une butée-guide (6) sont fixées dans des positions opposées aux faces du second élément (7), et
- le premier élément élastique (4) comporte en son centre un trou traversant (5) ayant un diamètre plus petit que celui du second élément (7), le second élément (7) changeant de place par rapport au premier élément (4) lorsqu'il passe à travers le trou (5), le fonctionnement de la seringue étant tel que
- lorsque le piston (3) se trouve près du canon (2), ce qui est une condition nécessaire pour remplir la seringue, le second élément (7) se trouve entre l'extrémité du corps (1) et le premier élément (4),
- lorsque la tige du piston (3) est actionnée pour introduire le moyen d'injection dans la seringue, le second élément (7) est introduit dans le trou (5) du premier élément (4) de manière à former l'ensemble étanche à l'eau, et
- lorsque le piston (3) atteint le moyen (12) de limitation de course et la tige (9) continue d'être actionnée dans la même direction, le second élément (7) passe à travers le trou du premier élément (4), de sorte qu'il occupe une position à partir de laquelle il est possible d'appliquer une pression sur la tige (9), de manière que les éléments (4 et 7) agissent comme un piston et que le moyen d'injection soit expulsé à travers le canon (2) jusqu'à l'extrémité de la course lorsque la butée-guide (6) touche le fond de la seringue, ceci ne permettant pas au premier élément (4) de passer à travers le trou (5).

2. Seringue selon la revendication 1, dans laquelle le second élément (7) a la forme d'un cône tronqué (8) dont la petite base est fixée à la tige (9) et la grande base est fixée à la butée-guide (6).

3. Seringue selon la revendication 1, dans laquelle le second élément (7) a une forme sphérique (10), et la butée-guide (6) ainsi que la tige (9) sont fixées à des endroits diamétralement opposés de la sphère.

4. Seringue selon la revendication 1, dans laquelle le second élément (7) est formé par un hémisphère (11) où la tige (9) est appliquée dans sa partie sphérique et la butée-guide (6) se trouve dans la partie plate.

5. Seringue selon la revendication 1, dans laquelle le second élément (7) a des configurations géométriques polygonales.

6. Seringue selon la revendication 1, dans laquelle les moyens (12) de limitation de course sont formés par des bosses sur la paroi du tube constituant le corps (1) de la seringue à 120° les unes des autres.

7. Seringue selon la revendication 6, dans laquelle les moyens (12) de limitation de course sont formés par des déformations ayant une forme courbée sur les parois du tube.

8. Seringue selon la revendication 6, dans laquelle les moyens (12) de limitation de course sont formés par des déformations rectangulaires sur les parois du tube.

9. Seringue selon la revendication 6, dans laquelle les moyens (12) de limitation de course sont formés par une bague, liée de façon solidarisée, au corps (1) de la seringue contenant la tige (9).

10. Seringue selon la revendication 6, dans laquelle les moyens (12) de limitation de course sont formés par une déformation de l'extrémité du tube de la seringue.
